# DEMANDE DE BREVET EUROPEEN

(11) **EP 2 952 227 A1**
(43) Date de publication de la demande: **09.12.2015**
(21) Numéro de dépôt: 15170599.3
(22) Date de dépôt: 03.06.2015
(51) Int. Cl.: A61N 5/10

(54) **OBJET-TEST POUR CORRECTION DES MOUVEMENTS PARASITES D'IMAGEUR DE SORTIE EQUIPANT UN APPAREIL DE TRAITEMENT PAR RADIOTHERAPIE EXTERNE LORSQUE CELUI-CI EST EN MOUVEMENT**

(30) Priorité: 03.06.2014 FR 1455040
(71) Demandeur: Qualiformed, 85000 La Roche-sur-Yon (FR)
(72) Inventeur: Beaumont, Stéphane, 85430 Nieul le Dolent (FR)
(74) Mandataire: Cabinet Chaillot

(57) **Abrégé**

La présente invention porte sur un objet-test (20) pour un appareil de traitement par radiothérapie externe, ledit appareil de traitement par radiothérapie externe comprenant un statif rotatif portant à une extrémité une tête d'irradiation contenant une source de rayonnement et à l'autre extrémité un imageur de sortie, ledit objet-test (20) étant destiné à corriger les mouvements parasites, vis-à-vis de la source de rayonnement, de l'imageur de sortie lors d'un mouvement de l'appareil de traitement par radiothérapie externe, caractérisé par le fait que ledit objet-test (20) comprend une plaque radiotransparente (2) dans l'épaisseur de laquelle est noyé au moins un élément radio-opaque (3a, 3b, 3c, 3d; 11a, 11b), ledit ou lesdits éléments radio-opaques formant un motif géométrique ayant au moins deux points et le centre dudit motif géométrique correspondant au centre de ladite plaque radiotransparente (2), et des moyens de fixation permettant la fixation de l'objet-test (20) sur la tête d'irradiation.

## Description

La présente invention concerne le domaine du traitement par radiothérapie externe, et porte en particulier sur des objets-test et sur des procédés de correction des mesures réalisées dans les images correspondant aux tests de performance d'un appareil de traitement par radiothérapie externe pour prendre en compte des mouvements parasites, vis-à-vis de la source de rayonnement de l'appareil de traitement, d'un imageur de sortie équipant ledit appareil de traitement par radiothérapie externe lorsque le statif de celui-ci est en mouvement.

Dans un traitement par radiothérapie externe, on stérilise les tumeurs en les irradiant à haute dose à l'aide de multiples faisceaux de rayonnements ionisants (le plus souvent des rayons X, mais on utilise également d'autres rayonnements tels que des faisceaux d'électrons, de neutrons, d'ions), concentriques ou non. Le positionnement du patient par rapport aux faisceaux d'irradiation est primordial : en effet, un mauvais positionnement du patient par rapport aux faisceaux d'irradiation se traduira d'une part par une irradiation incomplète de la tumeur, ce qui peut induire une récidive de la maladie, et d'autre part par une irradiation des tissus sains avoisinant la tumeur, ce qui peut provoquer des graves complications pour le patient.

Pour garantir un ciblage des faisceaux d'irradiation sur la tumeur, plusieurs tests de contrôle de qualité des appareils de traitement par radiothérapie externe ont été mis au point, lesquels sont effectués sur l'appareil de traitement par radiothérapie externe avant le traitement par radiothérapie externe d'un patient.

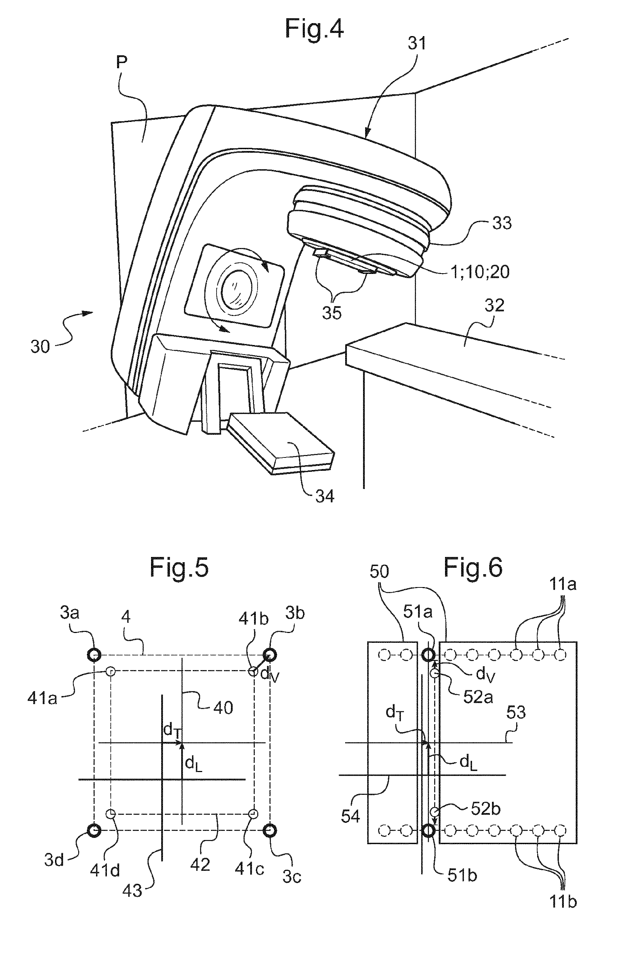

Un appareil de traitement par radiothérapie externe classique, représenté par exemple sur la Figure 4, comprend un statif rotatif, portant à une extrémité une tête d'irradiation portant une source de rayonnement, la tête d'irradiation se terminant par un collimateur qui permet de délimiter le faisceau d'irradiation, et à l'autre extrémité un imageur de sortie appelé « imageur portal » qui permet de faire des radiographies numériques d'un objet placé entre le collimateur et l'imageur de sortie, en général sur une table de traitement, également appelée support patient. L'imageur de sortie est donc utilisé soit pour contrôler la position du patient avant de le traiter par radiothérapie externe, soit pour réaliser des tests de performance de l'appareil de traitement par radiothérapie externe.

De tels tests de performance de l'appareil de traitement par radiothérapie externe sont par exemple décrits dans le rapport *"Quality Assurance of Medical accelerators"* (Assurance qualité d'accélérateurs médicaux) du groupe de travail (task group) 142 de l'American Association of Physicists In Medicine (AAPM) (Association Américaine des Physiciens en Médecine) (TG 142 de l'AAPM).

La tête d'irradiation de l'appareil de traitement par radiothérapie externe projette en direction du support patient puis de l'imageur de sortie un faisceau d'irradiation destiné au traitement du patient, lequel passe à travers le collimateur qui est la partie finale de la tête d'irradiation et qui permet de délimiter le faisceau d'irradiation selon de nombreuses configurations.

Lorsque le statif de l'appareil de traitement par radiothérapie externe est en rotation, la tête de l'appareil qui contient la source de rayonnement et le collimateur étant en porte-à-faux, son poids élevé fait que la source de rayonnement avance longitudinalement (dans le cas où la tête est en haut) ou recule longitudinalement (dans le cas où la tête est en bas) par rapport à un plan vertical perpendiculaire à l'axe de rotation du statif de l'appareil de traitement par radiothérapie externe. L'imageur de sortie situé sur le statif à l'opposé de la source de rayonnement subit des contraintes mécaniques du même type, mais d'intensités très différentes en raison du fait que l'imageur de sortie est beaucoup plus léger que la tête de l'appareil de traitement par radiothérapie externe, les mouvements parasites longitudinaux et verticaux de l'imageur de sortie vis-à-vis de l'axe du faisceau d'irradiation étant dus en grande partie à la flexion du statif sous l'effet du poids conjugué de la tête d'irradiation et de l'imageur de sortie. Le mouvement parasite vertical exprime le fait que la source de rayonnement n'est pas à une distance constante de l'imageur de sortie lorsque le statif est en rotation. Les mouvements parasites longitudinaux et transversaux expriment le fait que l'axe du faisceau d'irradiation ne se projette pas strictement au même endroit sur l'imageur de sortie lorsque le statif est en rotation.

De plus, lorsque le statif est en rotation, l'imageur de sortie qui a généralement la possibilité de se déplacer dans les trois dimensions de l'espace grâce à des moteurs, des engrenages et des vis sans fin, présente des jeux mécaniques dont la plus forte influence porte sur des déplacements parasites transversaux de l'imageur de sortie vis-à-vis de l'axe du faisceau d'irradiation.

Ainsi, lorsque le statif est en rotation, la distance source de rayonnement-imageur de sortie n'est pas constante et l'alignement longitudinal ainsi que l'alignement transversal de l'imageur de sortie par rapport à la source de rayonnement varient, les valeurs typiques d'intensité de ces mouvements parasites étant, pour un appareil de traitement très performant, de l'ordre de 1 mm dans la direction longitudinale, 1 mm dans la direction transversale et 2 mm dans la direction verticale.

Il est donc nécessaire de corriger les mouvements parasites de l'imageur de sortie lorsque celui-ci est en mouvement avec la rotation du statif de l'appareil de traitement par radiothérapie externe, dans le cadre de certains contrôles de performances du collimateur multi-lames (MLC) utilisé pour à la fois délimiter le faisceau d'irradiation et moduler son intensité durant le traitement.

La demande de brevet français FR2956979 décrit un objet-test et un procédé utilisant cet objet-test permettant de contrôler la coïncidence des champs lumineux et des champs irradiés sur un appareil de traitement par radiothérapie externe, l'objet-test étant constitué d'un corps d'une certaine densité électronique et d'au moins un élément intégré sur une surface du corps ou noyé dans celui-ci et apte à être distingué du corps par imagerie par faisceaux de rayonnements ionisants. Cependant cet objet-test ne permet pas de mesurer avec précision les mouvements parasites de l'imageur de sortie de l'appareil de traitement par radiothérapie externe lorsque celui-ci est en mouvement avec la rotation du statif, afin de corriger lesdits mouvements parasites de l'imageur de sortie.

La présente invention vise à résoudre les inconvénients de l'état de la technique, en proposant un objet-test servant à mesurer avec précision les mouvements parasites de l'imageur de sortie d'un appareil de traitement par radiothérapie externe lorsque celui-ci est en mouvement avec la rotation du statif de l'appareil de traitement par radiothérapie externe, afin de corriger lesdits mouvements parasites de l'imageur de sortie en fonction de l'angle de rotation du statif, ledit objet-test comprenant une plaque radiotransparente dans laquelle plusieurs éléments radio-opaques sont noyés dans l'épaisseur de la plaque radiotransparente, l'objet-test étant fixé au niveau de la sortie de la tête d'irradiation de l'appareil de traitement par radiothérapie externe, et au plus près de celle-ci, l'objet-test ayant de plus une structure simple et donc facile à produire et peu onéreuse.

La présente invention propose également deux procédés de mesure et de correction des mesures réalisées dans les images correspondant aux tests de performance d'un appareil de traitement par radiothérapie externe pour prendre en compte des mouvements parasites d'un imageur de sortie équipant un appareil de traitement par radiothérapie externe lorsque celui-ci est en mouvement, les deux procédés utilisant l'objet-test selon la présente invention, le premier procédé nécessitant un prétest pour mesurer les corrections qui seront à réaliser pendant le test de performance de l'appareil de traitement par radiothérapie externe et le second procédé réalisant les mesures des corrections à réaliser directement pendant le test de performance de l'appareil de traitement par radiothérapie externe.

La présente invention porte donc sur un objet-test pour un appareil de traitement par radiothérapie externe, ledit appareil de traitement par radiothérapie externe comprenant un statif rotatif portant à une extrémité une tête d'irradiation contenant une source de rayonnement et à l'autre extrémité un imageur de sortie de type imageur portal, ledit objet-test étant destiné à corriger les mouvements parasites, vis-à-vis de la source de rayonnement de l'appareil de traitement par radiothérapie externe, de l'imageur de sortie lors d'un mouvement de l'appareil de traitement par radiothérapie externe, caractérisé par le fait que ledit objet-test comprend une plaque radiotransparente dans l'épaisseur de laquelle est noyé au moins un élément radio-opaque, ledit ou lesdits éléments radio-opaques formant un motif géométrique ayant au moins deux points et le centre dudit motif géométrique correspondant au centre de ladite plaque radiotransparente, et des moyens de fixation permettant la fixation de l'objet-test sur la tête d'irradiation.

Ainsi, l'objet-test possède une structure simple et peut donc être fabriqué à un faible coût.

Le ou les éléments radio-opaques peuvent être un fil, une bille, ou tout élément de petite dimension apte à être noyé dans l'épaisseur de la plaque radiotransparente.

Par l'adjectif « radiotransparente », on entend que la plaque est radiotransparente ou quasi radiotransparente, laissant passer la majeure partie du rayonnement. La plaque radiotransparente laisse ainsi passer les rayonnements de haute énergie produits par l'appareil de traitement par radiothérapie externe, avec une atténuation maximale de 10% de l'intensité du rayonnement après passage à travers la plaque radiotransparente.

Le motif géométrique est formé dans le plan de la plaque radiotransparente et peut être un segment de deux points, un triangle, un carré, un rectangle, un polygone régulier, ou tout motif géométrique dont le centre peut être facilement déterminé géométriquement. Il est à noter que le motif géométrique peut être continu (un fil noyé en boucle fermée dans l'épaisseur de la plaque radiotransparente et formant ledit motif géométrique) ou discret (des billes placées à des sommets du motif géométrique).

Le ou les éléments radio-opaques permettent de déterminer précisément la position de l'image de ceux-ci sur l'imageur de sortie après irradiation de l'objet-test par une source de rayonnement.

Le ou les éléments radio-opaques peuvent, de préférence, être situés au niveau d'au moins deux des bords de la plaque radiotransparente afin de ne pas obstruer la partie centrale de l'image utile dans le cadre du test de performance de l'appareil de traitement par radiothérapie externe.

De préférence, les éléments radio-opaques sont tous noyés dans l'épaisseur de la plaque radiotransparente à une même profondeur par rapport à la face de la plaque radiotransparente en regard de la tête d'irradiation, lesdits éléments radio-opaques étant ainsi tous disposés à la même distance de la source de rayonnement de la tête d'irradiation.

Les moyens de fixation permettent une fixation de l'objet-test sur la tête d'irradiation, et donc une solidarisation de l'objet-test à la tête d'irradiation, permettant ainsi de parfaitement caractériser les changements relatifs de position entre la tête d'irradiation (donc la source de rayonnement) et l'imageur de sortie lors d'un mouvement de l'appareil de traitement par radiothérapie externe.

L'objet-test est avantageusement fixé immédiatement en sortie de la tête d'irradiation, pour bouger solidairement avec celle-ci.

Les moyens de fixation peuvent être constitués par un cadre métallique creux sur lequel la plaque radiotransparente est fixée, ledit cadre étant adapté afin d'être inséré dans une paire de glissières disposées au niveau de la sortie de la tête d'irradiation de l'appareil de traitement par radiothérapie externe, l'objet-test étant ainsi au plus près de la source de rayonnement de la tête d'irradiation.

Sans s'écarter cependant du cadre de la présente invention, les moyens de fixation peuvent être des vis se vissant dans la tête d'irradiation, un adhésif, ou plus généralement tout moyen permettant de solidariser, de préférence de manière amovible, l'objet-test à la tête d'irradiation.

Selon un premier mode de réalisation de l'invention, la plaque radiotransparente porte au moins deux billes radio-opaques.

Ainsi, l'objet-test selon le premier mode de réalisation possède une structure simple et est donc facile à produire.

De plus, de nombreuses géométries peuvent convenir pour le placement des billes dans la plaque radiotransparente, telles qu'une ligne formée par deux billes, un triangle équilatéral formé par trois billes, ou un carré, un rectangle.

Selon un second mode de réalisation de l'invention, la plaque radiotransparente porte deux rangées parallèles de billes, chaque rangée de billes comprenant au moins deux billes, les deux rangées de billes comprenant le même nombre de billes espacées du même pas, de telle sorte que la ligne joignant les billes en regard des deux rangées est perpendiculaire à la direction des rangées de billes.

Un motif en « échelle » est ainsi formé, chaque rangée constituant l'un des montants de l'échelle, et chaque bille représentant l'une des extrémités d'un barreau de l'échelle.

Ainsi, bien que l'objet-test selon le second mode de réalisation soit plus complexe et donc plus onéreux à produire que l'objet-test selon le premier mode de réalisation, cet objet-test selon le second mode de réalisation possède tout de même une structure relativement simple.

Les deux rangées de billes parallèles sont adaptées pour un appareil de traitement par radiothérapie externe comprenant un collimateur qui possède deux bancs de lames permettant de décrire une fente glissante longitudinale en fonction de l'angle de rotation du statif, seule une paire de billes des deux rangées de billes parallèles étant visible par la source de rayonnement pour un angle de rotation de statif donné.

Selon un troisième mode de réalisation de l'invention, l'objet-test comprend en outre au moins quatre billes radio-opaques formant un carré ou un rectangle dont le contour entoure les deux rangées de billes, le centre du carré ou rectangle formé par les quatre billes radio-opaques correspondant au barycentre des deux rangées de billes, c'est-à-dire à l'intersection des diagonales du rectangle dont les sommets sont les quatre billes d'extrémité des deux rangées de billes.

L'objet-test selon le troisième mode de réalisation est une combinaison des objets-test des premier et second modes de réalisation.

Selon une caractéristique particulière de l'invention, la plaque radiotransparente est en matière plastique radio-transparente, qui lui assure une radio-transparence ou quasi radio-transparence aux rayonnements de haute énergie produits par l'appareil de traitement par radiothérapie externe et une bonne stabilité dimensionnelle sous l'effet répété de ces rayonnements. La matière plastique radio-transparente est, de préférence, du polycarbonate.

Ainsi, la plaque radiotransparente est peu onéreuse, est facile à perforer pour y insérer les éléments radio-opaques, et contrairement aux éléments radio-opaques est radiotransparente afin de laisser passer à travers celle-ci les rayons ionisants émis par la source de rayonnement de la tête d'irradiation de l'appareil de traitement par radiothérapie externe.

Selon une caractéristique particulière de l'invention, l'épaisseur de la plaque radiotransparente est constante et comprise entre 3 et 10 mm, de préférence 6 mm.

Ainsi, l'épaisseur de la plaque radiotransparente étant constante, les rayons ionisants émis par la source de rayonnement traversent tous sensiblement la même épaisseur de plaque radiotransparente.

De plus, la plage d'épaisseurs allant de 3 à 10 mm permet à la plaque radiotransparente d'être suffisamment rigide pour ne pas se déformer quand le statif tourne et de n'être pas trop épaisse afin de garder un bon contraste sur les images pour les billes radio-opaques qu'elle englobe.

Selon une caractéristique particulière de l'invention, les éléments radio-opaques sont en carbure de tungstène, mais peuvent également être en tout autre métal ou en tout autre matériau de densité électronique nettement supérieure, notamment au moins 2 fois supérieure, à celle de la plaque radiotransparente.

Ainsi, les éléments radio-opaques possèdent de bonnes propriétés de radio-opacité, et sont donc bien repérables sur l'image formée, permettant un traitement informatique de l'image formée.

Il est à noter que la forme de bille est la forme préférée pour les éléments radio-opaques car la forme sphérique de la surface assure que l'image par le faisceau d'irradiation des éléments radio-opaques détectée par l'imageur de sortie est la même, quelles que soient les conditions de divergence du faisceau d'irradiation et donc quelle que soit la largeur du faisceau d'irradiation.

Selon une caractéristique particulière de l'invention, chaque bille radio-opaque est noyée dans la plaque radiotransparente de telle sorte que son centre soit à une distance de la face de la plaque radiotransparente sensiblement égale à son rayon.

Ainsi, toutes les billes sont à la même profondeur par rapport à la face de la plaque radiotransparente en regard de la tête d'irradiation et donc sont toutes à la même distance de la source de rayonnement de la tête d'irradiation.

Le diamètre des billes est assez gros pour que les images de billes après irradiation puissent être vues par l'imageur de sortie de l'appareil de traitement par radiothérapie externe. Ainsi, le diamètre des billes peut être compris entre 1 et 10 mm.

Selon le premier mode de réalisation, le diamètre des billes est compris entre 2 et 6 mm, et peut avantageusement être de 4 mm.

Selon le deuxième mode de réalisation, le diamètre des billes est compris entre 1 et 5 mm, et peut avantageusement être de 2 mm.

Selon le troisième mode de réalisation, le diamètre des quatre billes formant un carré ou un rectangle entourant les deux rangées de billes est compris entre 2 et 6 mm, et peut avantageusement être de 4 mm.

Ainsi, toutes les billes des deux rangées de billes sont à la même profondeur par rapport à la surface supérieure de la plaque radiotransparente en regard avec la tête d'irradiation et donc sont toutes à la même distance de la source de rayonnement de la tête d'irradiation.

De plus, le diamètre des billes des deux rangées de billes est assez gros pour que les images de billes après irradiation puissent être vues par l'imageur de sortie de l'appareil de traitement par radiothérapie externe, le diamètre des billes étant également suffisamment petit pour qu'une bille puisse être contenue dans la fente d'irradiation du collimateur de l'appareil de traitement par radiothérapie externe.

Selon une caractéristique particulière de l'invention, la plaque radiotransparente comporte un réticule gravé au niveau de la face de celle-ci destinée à être en regard de la tête d'irradiation, ledit réticule représentant le centre de la plaque radiotransparente.

Ainsi, le réticule gravé sur la plaque radiotransparente permet de repérer visuellement le centre de la plaque radiotransparente, ce qui est utile lors de l'installation de l'objet-test sur l'appareil de traitement par radiothérapie externe par un utilisateur.

Avantageusement, chaque élément radio-opaque d'un objet-test selon la présente invention est à une distance minimale du centre de la plaque radiotransparente comprise entre 50 mm et 70 mm, de préférence de 62 mm.

On s'assure ainsi qu'aucun élément radio-opaque n'apparaîtra dans la partie utile (centrale) de l'image sur l'imageur de sortie, tout en s'assurant que chaque élément radio-opaque soit dans le faisceau de rayonnement, pour être visible sur les bords de l'image formée sur l'imageur de sortie, afin de pouvoir effectuer des mesures sur le motif géométrique formé dans l'image de l'imageur de sortie par le ou les éléments radio-opaques.

L'invention a également pour objet un procédé avec prétest de mesure et de correction des mesures réalisées dans les images correspondant aux tests de performance d'un appareil de traitement par radiothérapie externe pour prendre en compte les mouvements parasites, vis-à-vis de la source de rayonnement de l'appareil de traitement par radiothérapie externe, d'un imageur de sortie équipant ledit appareil de traitement par radiothérapie externe lorsque celui-ci est en mouvement, l'appareil de traitement par radiothérapie externe comprenant en outre un statif rotatif portant à une extrémité une tête d'irradiation contenant la source de rayonnement et à l'autre extrémité un imageur de sortie, caractérisé par le fait qu'il comprend les étapes consistant à :
- fixer un objet-test tel que défini ci-dessus sur la tête d'irradiation de l'appareil de traitement par radiothérapie externe, l'objet-test étant fixé très fermement à une certaine distance de la source de rayonnement de la tête d'irradiation et au plus près de celle-ci ;
- réaliser un prétest consistant à :
   - irradier l'objet-test avec un faisceau de rayonnement émis à partir de la tête d'irradiation de l'appareil de traitement par radiothérapie externe pendant une séquence d'angles de rotation du statif de l'appareil de traitement par radiothérapie externe ;
   - acquérir une succession d'images de l'objet-test sur l'imageur de sortie en fonction de la séquence d'angles de rotation du statif de l'appareil de traitement par radiothérapie externe ;
   - mesurer la taille du motif géométrique formé par les éléments radio-opaques de l'objet-test et la position du centre dudit motif dans toutes les images acquises, à l'aide des marques créées par les éléments radio-opaques sur les images après irradiation de l'objet-test ;
   - comparer la taille réelle du motif géométrique formé par les éléments radio-opaques de l'objet-test à la taille mesurée du motif géométrique formé par les éléments radio-opaques de l'objet-test sur chaque image afin d'obtenir la distance source de rayonnement-imageur de sortie (DSI) et donc la mesure des mouvements verticaux parasites de l'imageur de sortie en fonction de l'angle de rotation du statif ; et
   - comparer la position du centre de l'imageur de sortie à la position mesurée du centre du motif géométrique formé par les éléments radio-opaques de l'objet-test sur chaque image afin d'obtenir la mesure des mouvements longitudinaux et transversaux parasites de l'imageur de sortie en fonction de l'angle de rotation du statif, la mesure étant corrigée des variations de DSI calculées précédemment ;
- retirer l'objet-test de la tête d'irradiation ;
- acquérir les images correspondant au test de performance de l'appareil de traitement par radiothérapie externe ; et
- corriger les mesures réalisées dans ces images et caractérisant les performances de l'appareil de traitement par radiothérapie externe, de tous les mouvements parasites de l'imageur de sortie à l'aide des mesures réalisées lors du prétest.

Ce premier procédé est donc avantageusement utilisé avec un objet-test selon le premier ou le troisième mode de réalisation.

Les images acquises lors du prétest selon le premier procédé peuvent être acquises en mode statique ou en mode cinéma (images produites à intervalles réguliers pendant toute la rotation du statif).

L'invention a également pour objet un procédé sans prétest de mesure et de correction des mesures réalisées dans les images correspondant aux tests de performance d'un appareil de traitement par radiothérapie externe pour prendre en compte les mouvements parasites, vis-à-vis de la source de rayonnement de l'appareil de traitement par radiothérapie externe, d'un imageur de sortie équipant ledit appareil de traitement par radiothérapie externe lorsque celui-ci est en mouvement, l'appareil de traitement par radiothérapie externe comprenant en outre un statif rotatif portant à une extrémité une tête d'irradiation contenant la source de rayonnement qui se termine par un collimateur permettant de délimiter un faisceau de rayonnement et à l'autre extrémité un imageur de sortie, caractérisé par le fait qu'il comprend les étapes consistant à :
- fixer un objet-test tel que défini ci-dessus sur la tête d'irradiation de l'appareil de traitement par radiothérapie externe, l'objet-test étant fixé très fermement à une certaine distance de la source de rayonnement de la tête d'irradiation et au plus près de celle-ci ;
- irradier l'objet-test lors du test de performance de l'appareil de traitement par radiothérapie externe avec un faisceau de rayonnement émis à partir de la tête d'irradiation de l'appareil de traitement par radiothérapie externe pendant une séquence d'angles de rotation du statif de l'appareil de traitement par radiothérapie externe, le collimateur délimitant une fente glissante glissant de manière transversale réalisée par ses deux bancs de lames transversalement opposés afin de n'irradier qu'une seule colonne de billes des deux rangées de billes parallèles pour un angle de rotation donné du statif ;
- acquérir l'image de l'objet-test sur l'imageur de sortie pendant toute la séquence d'angles de rotation du statif, chaque colonne de l'image ayant été acquise pour un angle de rotation du statif donné ;
- mesurer dans l'image acquise la distance longitudinale séparant dans chaque colonne de l'image la paire de billes correspondant à l'angle de rotation de statif associé et la position du centre de la paire de billes correspondant à l'angle de rotation de statif associé, à l'aide des marques créées par les billes des deux rangées de billes parallèles sur l'image après irradiation de l'objet-test ;
- dans l'image acquise, comparer la distance réelle séparant les deux rangées de billes à la distance mesurée séparant la paire de billes correspondant à l'angle de rotation de statif associé, afin d'obtenir la distance source de rayonnement-imageur de sortie (DSI) et donc la mesure des mouvements verticaux parasites de l'imageur de sortie en fonction de l'angle de rotation du statif ;
- dans l'image acquise, comparer la position réelle du centre de la paire de billes correspondant à l'angle de rotation de statif associé à la position mesurée du centre de la paire de billes correspondant à l'angle de rotation de statif associé, afin d'obtenir la mesure des mouvements longitudinaux et transversaux parasites de l'imageur de sortie en fonction de l'angle de rotation du statif, la mesure étant corrigée des variations de DSI calculées précédemment ; et
- dans l'image acquise, correspondant également au test de performance de l'appareil de traitement par radiothérapie externe, corriger les mesures réalisées dans l'image et caractérisant les performances de l'appareil de traitement par radiothérapie externe, de tous les mouvements parasites de l'imageur de sortie à l'aide des mesures réalisées précédemment.

Ce deuxième procédé est avantageusement utilisé avec un objet-test selon le deuxième ou troisième mode de réalisation.

L'image acquise selon le deuxième procédé est acquise en mode intégration (image produite correspond au cumul du rayonnement produit pendant tout le mouvement de rotation du statif).

Ainsi le procédé avec prétest prend en compte indirectement les mouvements parasites de l'imageur de sortie, vis-à-vis de la source de rayonnement de l'appareil de traitement par radiothérapie externe, alors que le procédé sans prétest prend en compte directement ces mêmes mouvements parasites.

Le collimateur se compose de deux bancs transversalement opposés portant chacun de multiples lames permettant de délimiter des faisceaux de rayonnement de forme complexe. Ainsi dans le procédé sans prétest, le collimateur délimite, par ses deux bancs de lames transversalement opposés, une fente glissante qui glisse de manière transversale pendant le mouvement de rotation du statif de l'appareil de traitement par radiothérapie externe.

Pour mieux illustrer l'objet de la présente invention, on va en décrire ci-après, à titre illustratif et non limitatif, trois modes de réalisation préférés, avec référence aux dessins annexés.

Sur ces dessins :
- la Figure 1 est une vue de dessous d'un objet-test selon un premier mode de réalisation de la présente invention ;
- la Figure 2 est une vue de dessous d'un objet-test selon un second mode de réalisation de la présente invention ;
- la Figure 3 est une vue de dessous d'un objet-test selon un troisième mode de réalisation de la présente invention ;
- la Figure 4 est une vue en perspective d'un appareil de traitement par radiothérapie externe équipé d'un objet-test selon la présente invention ;
- la Figure 5 est une vue schématique de la superposition de la vue de dessus de l'objet-test du premier mode de réalisation de la présente invention et de l'image associée capturée par l'imageur de sortie de l'appareil de traitement par radiothérapie externe à un angle de rotation de statif donné ; et
- la Figure 6 est une vue schématique de la superposition de la vue de dessus de l'objet-test du second mode de réalisation de la présente invention et de l'image associée capturée par l'imageur de sortie de l'appareil de traitement par radiothérapie externe à un angle de rotation de statif donné.

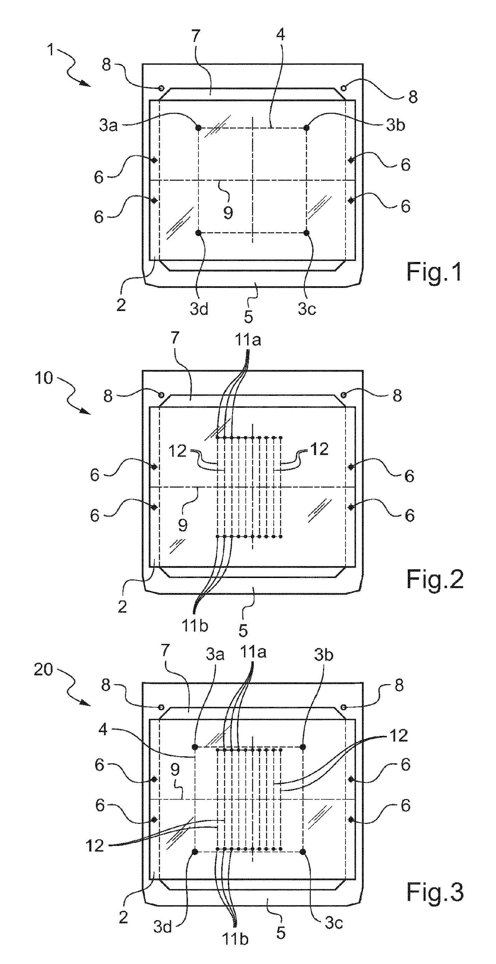

Si l'on se réfère à la Figure 1, on peut voir qu'il y est représenté un objet-test 1 selon un premier mode de réalisation de l'invention.

L'objet-test 1 comporte une plaque radiotransparente 2 dans l'épaisseur de laquelle sont noyés quatre éléments radio-opaques 3a, 3b, 3c, 3d.

Les éléments radio-opaques 3a, 3b, 3c, 3d sont quatre billes métalliques disposées afin de former un carré 4 (représenté en pointillés sur la Figure 1 à titre illustratif) sur la plaque radiotransparente 2, les quatre éléments radio-opaques 3a, 3b, 3c, 3d étant tous à la même profondeur dans l'épaisseur de la plaque radiotransparente 2, deux des éléments radio-opaques 3a, 3b étant disposés au niveau de l'un des bords de la plaque radiotransparente 2 et les deux autres éléments radio-opaques 3c, 3d étant disposés au niveau du bord opposé de la plaque radiotransparente 2.

La plaque radiotransparente 2 est en matière plastique radio-transparente, de préférence du polycarbonate.

Il est à noter que la plaque radiotransparente 2 pourrait également être en carbone ou en tout autre matériau suffisamment rigide et de densité électronique nettement inférieure à celle des éléments radio-opaques 3a, 3b, 3c, 3d, notamment de densité électronique 2 fois inférieure à celle des éléments radio-opaques 3a, 3b, 3c, 3d, sans s'écarter du cadre de la présente invention.

L'épaisseur de la plaque radiotransparente 2 est constante et égale à 6 mm.

Il est à noter que l'épaisseur de la plaque radiotransparente 2 pourrait être comprise entre 3 et 10 mm, sans s'écarter du cadre de la présente invention, l'épaisseur devant être suffisante pour que la plaque radiotransparente 2 soit suffisamment rigide pour ne pas se déformer lors de la rotation du statif sur lequel elle est fixée, tout en étant suffisamment radiotransparente.

Les éléments radio-opaques 3a, 3b, 3c, 3d sont en carbure de tungstène.

Il est à noter que les éléments radio-opaques 3a, 3b, 3c, 3d pourraient également être en tout autre métal ou en tout autre matériau de densité électronique nettement supérieure à celle de la plaque radiotransparente 2, notamment de densité électronique 2 fois supérieure à celle de la plaque radiotransparente 2, sans s'écarter du cadre de la présente invention.

A titre indicatif, le rapport de densité électronique entre la plaque radiotransparente 2 et les éléments radio-opaques 3a, 3b, 3c, 3d est de préférence comprise entre 2 et 20, de la manière que l'on préfère le plus entre 10 et 20.

Le diamètre des éléments radio-opaques 3a, 3b, 3c, 3d est égal à 4 mm, le centre de chacun des éléments radio-opaques 3a, 3b, 3c, 3d étant à une profondeur, par rapport à la face supérieure de la plaque radiotransparente 2 destinée à être en regard de la tête d'irradiation de l'appareil de traitement par radiothérapie externe, égale au rayon de chaque élément radio-opaque 3a, 3b, 3c, 3d dans l'épaisseur de la plaque radiotransparente 2. Les éléments radio-opaques 3a, 3b, 3c, 3d affleurent donc sensiblement ladite face supérieure de la plaque radiotransparente 2.

Il est à noter que le diamètre des éléments radio-opaques 3a, 3b, 3c, 3d pourrait être compris entre 1 et 10 mm, sans s'écarter du cadre de la présente invention. L'épaisseur de la plaque radiotransparente 2 sera alors adaptée en conséquence.

L'objet-test 1 comporte en outre, dans le mode de réalisation représenté, un cadre métallique creux 5 comprenant quatre bords sur lequel la plaque radiotransparente 2 est fixée, la plaque radiotransparente 2 étant fixée sur le cadre métallique creux 5 par appui latéral de sa face supérieure au niveau de deux bords opposés du cadre métallique creux 5 par l'intermédiaire de quatre vis 6 passant à travers des trous correspondants réalisés sur la plaque radiotransparente 2 et le cadre métallique creux 5.

Le cadre métallique creux 5 comporte un trou 7 en son centre dimensionné de telle sorte que les éléments radio-opaques 3a, 3b, 3c, 3d soient à l'intérieur de ce trou 7 dans une vue de dessus de l'objet-test 1.

Les dimensions de longueur et de largeur de la plaque radiotransparente 2 sont respectivement inférieures à celles du cadre métallique creux 5, de telle sorte que la plaque radiotransparente 2 ne gêne pas l'introduction du cadre métallique creux 5 dans une paire de glissières (décrite plus en détail Figure 4) destinées à l'accueillir.

De préférence, la plaque radiotransparente 2 a une largeur de 200 mm et une longueur de 254 mm et le cadre métallique creux 5 a une largeur et une longueur autour de 250 mm, de préférence de 264 mm, le cadre métallique creux 5 étant adapté afin d'être inséré dans une paire de glissières disposées au niveau de la sortie de la tête d'irradiation d'un appareil de traitement par radiothérapie externe conventionnel. Les dimensions indiquées ci-dessus ne sont bien entendu pas limitatives, et l'homme du métier saura adapter les dimensions de la plaque aux dimensions de l'appareil de traitement par radiothérapie externe.

De préférence, les éléments radio-opaques 3a, 3b, 3c, 3d sont séparés d'une distance de 134 mm au niveau des arrêtes du carré 4, le carré 4 étant centré sur la plaque radiotransparente 2. Encore une fois, ces dimensions ne sont pas limitatives, et l'homme du métier saura adapter les dimensions pour que les éléments radio-opaques 3a, 3b, 3c, 3d soient visibles sur l'image (donc dans le champ du faisceau de rayonnement) tout en n'étant pas sur la partie centrale utile de l'image formée sur l'imageur de sortie.

Le cadre métallique creux 5 comporte en outre deux trous 8 au niveau de deux de ses angles afin de fixer fermement celui-ci dans la paire de glissières de l'appareil de traitement par radiothérapie externe conventionnel par l'intermédiaire de vis, après que l'objet-test 1 est complétement inséré dans la paire de glissières.

Tout autre mode de fixation de la plaque radiotransparente 2 sur la tête d'irradiation que le cadre métallique creux 5, permettant de fixer rigidement la plaque radiotransparente 2 à la tête d'irradiation, est envisageable dans le cadre de la présente invention.

La plaque radiotransparente 2 comporte en outre un réticule 9 gravé au niveau de la face supérieure de celle-ci, le réticule 9 représentant le centre de la plaque radiotransparente 2.

Il est à noter que les extérieurs de la plaque radiotransparente 2 sont, de préférence, soignés et non coupants, pour une manipulation sécurisée de celle-ci.

Si l'on se réfère à la Figure 2, on peut voir qu'il y est représenté un objet-test 10 selon un second mode de réalisation de l'invention.

L'objet-test 10 comporte une plaque radiotransparente 2 dans l'épaisseur de laquelle sont noyés deux rangées parallèles d'éléments radio-opaques 11a, 11b.

Les éléments radio-opaques sont deux rangées de billes métalliques parallèles 11a, 11b comprenant dans l'exemple représenté chacune dix billes métalliques (sans que ce nombre soit limitatif), l'espacement entre les dix billes de la première rangée de billes 11a étant constant et égal à l'espacement entre les dix billes de la seconde rangée de billes 11b, les dix billes de la première rangée de billes 11a étant alignées par rapport aux dix billes de la seconde rangée de billes 11b de telle sorte que les billes des première et seconde rangées de billes 11a, 11b sont alignées deux à deux parallèlement, dans une direction perpendiculaire à la direction des deux rangées, (des lignes 12 représentant chaque paire de billes des deux rangées de billes 11a, 11b sont représentées sur la Figure 2 à titre illustratif), les billes des deux rangées de billes 11a, 11b étant toutes à la même profondeur dans l'épaisseur de la plaque radiotransparente 2, la première rangée de billes 11a étant disposée au niveau de l'un des bords de la plaque radiotransparente 2 et la deuxième rangée de billes 11b étant disposée au niveau du bord opposé de la plaque radiotransparente 2.

Il est à noter que chaque rangée de billes 11a, 11b pourrait également comporter au moins deux billes, les deux rangées de billes 11a, 11b ayant le même nombre de billes, sans s'écarter du cadre de la présente invention.

La plaque radiotransparente 2 est en matière plastique radio-transparente, de préférence du polycarbonate.

Il est à noter que la plaque radiotransparente 2 pourrait également être en carbone ou en tout autre matériau de densité électronique nettement inférieure à celle des éléments radio-opaques 11a, 11b, notamment de densité électronique 2 fois inférieure à celle des éléments radio-opaques 11a, 11b, sans s'écarter du cadre de la présente invention. L'épaisseur de la plaque radiotransparente 2 est constante et égale à 6 mm.

Il est à noter que l'épaisseur de la plaque radiotransparente 2 pourrait être comprise entre 3 et 10 mm, sans s'écarter du cadre de la présente invention.

Les éléments radio-opaques 11a, 11b sont en carbure de tungstène.

Il est à noter que les éléments radio-opaques 11a, 11b pourraient également être en tout autre métal ou en tout autre matériau de densité électronique nettement supérieure à celle de la plaque radiotransparente 2, notamment de densité électronique 2 fois supérieure à celle de la plaque radiotransparente 2, sans s'écarter du cadre de la présente invention.

A titre indicatif, le rapport de densité électronique entre la plaque radiotransparente 2 et les éléments radio-opaques 11a, 11b est de préférence compris entre 2 et 20, de la manière que l'on préfère le plus entre 2 et 10.

Le diamètre des billes des deux rangées de billes 11a, 11b est égal à 2 mm, le centre de chacune des billes des deux rangées de billes 11a, 11b étant à une profondeur, par rapport à la face supérieure de la plaque radiotransparente 2, égale au rayon de chaque bille des deux rangées de billes 11a, 11b dans l'épaisseur de la plaque radiotransparente 2.

Il est à noter que le diamètre des billes des deux rangées de billes 11a, 11b pourrait être compris entre 1 et 10 mm, sans s'écarter du cadre de la présente invention. L'épaisseur de la plaque radiotransparente 2 sera alors adaptée en conséquence.

L'objet-test 10 comporte en outre un cadre métallique creux 5 comprenant quatre bords sur lequel la plaque radiotransparente 2 est fixée, la plaque radiotransparente 2 étant fixée sur le cadre métallique creux 5 par appui latéral de sa face supérieure destinée à être en regard de la tête d'irradiation au niveau de deux bords opposés du cadre métallique creux 5 par l'intermédiaire de quatre vis 6 passant à travers des trous correspondants réalisés sur la plaque radiotransparente 2 et le cadre métallique creux 5.

Le cadre métallique creux 5 comporte un trou 7 en son centre dimensionné de telle sorte que les billes des deux rangées de billes 11a, 11b soient à l'intérieur de ce trou 7 dans une vue de dessus de l'objet-test 10.

Les dimensions de longueur et de largeur de la plaque radiotransparente 2 sont respectivement inférieures à celles du cadre métallique creux 5 de telle sorte que la plaque radiotransparente 2 ne gêne pas l'introduction du cadre métallique creux 5 dans une paire de glissières destinées à l'accueillir.

De préférence, la plaque radiotransparente 2 a une largeur de 200 mm et une longueur de 254 mm et le cadre métallique creux 5 a une largeur et une longueur autour de 250 mm, de préférence de 264 mm, le cadre métallique creux 5 étant adapté afin d'être inséré dans une paire de glissières disposées au niveau de la sortie de la tête d'irradiation d'un appareil de traitement par radiothérapie externe conventionnel. Les dimensions indiquées ci-dessus ne sont bien entendu pas limitatives, et l'homme du métier saura adapter les dimensions de la plaque aux dimensions de l'appareil de traitement par radiothérapie externe.

De préférence, les deux rangées de billes 11a, 11b sont séparés d'une distance de 123,6 mm, les deux rangées de billes 11a, 11b étant sensiblement centrées sur la plaque radiotransparente 2. Encore une fois, ces dimensions ne sont pas limitatives, et l'homme du métier saura adapter les dimensions pour que les deux rangées de billes 11a, 11b soient visibles sur l'image (donc dans le champ du faisceau de rayonnement) tout en n'étant pas sur la partie centrale utile de l'image formée sur l'imageur de sortie.

De préférence, l'espacement entre les billes de chaque rangée de billes 11a, 11b est de 8,6 mm, cet espacement étant adapté pour une utilisation de l'objet-test 20 sur un appareil de traitement par radiothérapie externe conventionnel comportant un collimateur avec deux bancs de lames permettant de décrire une fente glissante (« picket fence ») longitudinale en fonction de l'angle de rotation du statif de l'appareil de traitement par radiothérapie externe, une seule paire de billes étant irradiée pour un angle de rotation de statif donné.

Le cadre métallique creux 5 comporte en outre deux trous 8 au niveau de deux de ses angles afin de fixer fermement celui-ci dans la paire de glissières de l'appareil de traitement par radiothérapie externe conventionnel par l'intermédiaire de vis, après que l'objet-test 10 est complétement inséré dans la paire de glissières.

La plaque radiotransparente 2 comporte en outre un réticule 9 gravé au niveau de la face supérieure de celle-ci, le réticule 9 représentant le centre de la plaque radiotransparente 2.

Il est à noter que les extérieurs de la plaque radiotransparente 2 sont soignés et non coupants, permettant une manipulation sécurisée de celle-ci.

Si l'on se réfère à la Figure 3, on peut voir qu'il y est représenté un objet-test 20 selon un troisième mode de réalisation de l'invention.

Il est à noter que cet objet-test 20 est une combinaison de l'objet-test 1 et de l'objet-test 10.

L'objet-test 20 comporte une plaque radiotransparente 2 dans l'épaisseur de laquelle sont noyés quatre éléments radio-opaques 3a, 3b, 3c, 3d et deux rangées parallèles d'éléments radio-opaques 11a, 11b.

Les quatre éléments radio-opaques 3a, 3b, 3c, 3d sont quatre billes métalliques disposées afin de former un carré 4 (représenté sur la Figure 3 à titre illustratif) sur la plaque radiotransparente 2, les quatre éléments radio-opaques 3a, 3b, 3c, 3d étant tous à la même profondeur dans l'épaisseur de la plaque radiotransparente 2, deux des éléments radio-opaques 3a, 3b étant disposés au niveau de l'un des bords de la plaque radiotransparente 2 et les deux autres éléments radio-opaques 3c, 3d étant disposés au niveau du bord opposé de la plaque radiotransparente 2.

Les deux rangées d'éléments radio-opaques 11a, 11b sont deux rangées de billes métalliques parallèles 11a, 11b identiques à celles du deuxième mode de réalisation. Leur description ne sera donc pas reprise en détail ici.

Les deux rangées de billes 11a, 11b sont disposées à l'intérieur du carré 4 formé par les quatre éléments radio-opaques 3a, 3b, 3c, 3d.

Il est à noter que les deux rangées de billes 11a, 11b pourraient être partiellement ou entièrement à l'extérieur du carré 4 formé par les quatre éléments radio-opaques 3a, 3b, 3c, 3d, sans s'écarter du cadre de la présente invention.

La plaque radiotransparente 2 est en matière plastique radio-transparente, de préférence du polycarbonate.

Il est à noter que la plaque radiotransparente 2 pourrait également être en carbone ou en tout autre matériau suffisamment rigide et de densité électronique nettement inférieure à celle des éléments radio-opaques 3a, 3b, 3c, 3d et 11a, 11b, notamment de densité électronique 2 fois inférieure à celle des éléments radio-opaques 3a, 3b, 3c, 3d et 11a, 11b, sans s'écarter du cadre de la présente invention.

L'épaisseur de la plaque radiotransparente 2 est constante et égale à 6 mm.

Il est à noter que l'épaisseur de la plaque radiotransparente 2 pourrait être comprise entre 3 et 10 mm, sans s'écarter du cadre de la présente invention, pour les mêmes raisons qu'indiqué ci-dessus en rapport avec les premier et deuxième modes de réalisation.

Les éléments radio-opaques 3a, 3b, 3c, 3d et 11a, 11b sont en carbure de tungstène.

Il est à noter que les éléments radio-opaques 3a, 3b, 3c, 3d et 11a, 11b pourraient également être en tout autre métal ou en tout autre matériau de densité électronique nettement supérieure à celle de la plaque radiotransparente 2, notamment de densité électronique 2 fois supérieure à celle de la plaque radiotransparente 2, sans s'écarter du cadre de la présente invention.

A titre indicatif, le rapport de densité électronique entre la plaque radiotransparente 2 et les éléments radio-opaques 3a, 3b, 3c, 3d et 11a, 11b est de préférence comprise entre 2 et 20, de la manière que l'on préfère le plus entre 2 et 10.

Le diamètre des quatre éléments radio-opaques 3a, 3b, 3c, 3d est égal à 4 mm, le centre de chacun des quatre éléments radio-opaques 3a, 3b, 3c, 3d étant à une profondeur, par rapport à la face supérieure de la plaque radiotransparente 2 en regard de la tête d'irradiation, égale au rayon de chaque élément radio-opaque 3a, 3b, 3c, 3d dans l'épaisseur de la plaque radiotransparente 2.

Il est à noter que le diamètre des quatre éléments radio-opaques 3a, 3b, 3c, 3d pourrait être compris entre 1 et 10 mm, sans s'écarter du cadre de la présente invention, l'épaisseur de la plaque radiotransparente étant alors adaptée en conséquence.

Le diamètre des billes des deux rangées de billes 11a, 11b est égal à 2 mm, le centre de chacune des billes des deux rangées de billes 11a, 11b étant à une profondeur, par rapport à la face supérieure de la plaque radiotransparente 2 en regard de la tête d'irradiation, égale au rayon de chaque bille des deux rangées de billes 11a, 11b dans l'épaisseur de la plaque radiotransparente 2.

Il est à noter que le diamètre des billes des deux rangées de billes 11a, 11b pourrait être compris entre 1 et 10 mm, sans s'écarter du cadre de la présente invention.

L'objet-test 20 comporte en outre un cadre métallique creux 5 identique à celui du deuxième mode de réalisation, et non décrit plus en détail ici.

Les dimensions de longueur et de largeur de la plaque radiotransparente 2 sont respectivement inférieures à celles du cadre métallique creux 5 de telle sorte que la plaque radiotransparente 2 ne gêne pas l'introduction du cadre métallique creux 5 dans une paire de glissières destinées à l'accueillir.

De préférence, la plaque radiotransparente 2 a une largeur de 200 mm et une longueur de 254 mm et le cadre métallique creux 5 a une largeur et une longueur autour de 250 mm, de préférence de 264 mm, le cadre métallique creux 5 étant adapté afin d'être inséré dans une paire de glissières disposées au niveau de la sortie de la tête d'irradiation d'un appareil de traitement par radiothérapie externe conventionnel. Les dimensions indiquées ci-dessus ne sont bien entendu pas limitatives, et l'homme du métier saura adapter les dimensions de la plaque aux dimensions de l'appareil de traitement par radiothérapie externe.

De préférence, les quatre éléments radio-opaques 3a, 3b, 3c, 3d sont séparés d'une distance de 134 mm au niveau des arrêtes du carré 4, le carré 4 étant centré sur la plaque radiotransparente 2.

De préférence, les deux rangées de billes 11a, 11b sont séparées d'une distance de 123.6 mm, les deux rangées de billes 11a, 11b étant sensiblement centrées sur la plaque radiotransparente 2.

De préférence, l'espacement entre les billes de chaque rangée de billes 11a, 11b est de 8,6 mm, cet espacement étant adapté pour une utilisation de l'objet-test 20 sur un appareil de traitement par radiothérapie externe conventionnel comportant un collimateur avec deux bancs de lames permettant de décrire une fente glissante (« picket fence ») longitudinale en fonction de l'angle de rotation du statif de l'appareil de traitement par radiothérapie externe, une seule paire de billes étant irradiée pour un angle de rotation de statif donné.

Le cadre métallique creux 5 comporte en outre deux trous 8 au niveau de deux de ses angles afin de fixer fermement celui-ci dans la paire de glissières de l'appareil de traitement par radiothérapie externe conventionnel par l'intermédiaire de vis, après que l'objet-test 20 est complétement inséré dans la paire de glissières.

La plaque radiotransparente 2 comporte en outre un réticule 9 gravé au niveau de la face supérieure de celle-ci, le réticule 9 représentant le centre de la plaque radiotransparente 2.

Il est à noter que les extérieurs de la plaque radiotransparente 2 sont soignés et non coupants, pour permettre sa manipulation sécurisée.

Si l'on se réfère à la Figure 4, on peut voir qu'il y est représenté un appareil de traitement par radiothérapie externe conventionnel 30 équipé d'un objet-test 1, 10, 20 selon la présente invention, dans une salle de traitement par radiothérapie externe.

Cet appareil 30 comporte, de manière classique, une structure qui comporte au moins une paroi verticale P portant un statif 31, et une table ou support patient 32.

Sur cette paroi verticale P est monté de manière classique mobile en rotation autour d'axe horizontal le statif 31, sensiblement en forme de U vu de profil. Le statif 31 porte à une extrémité une tête d'irradiation 33 qui se termine par un collimateur, et à l'autre extrémité, en face du collimateur de la tête d'irradiation 33 et tourné vers celui-ci, un imageur de sortie 34 capable de détecter un rayonnement émis à partir du collimateur de la tête d'irradiation 33, ledit imageur de sortie 34 permettant de réaliser des clichés du rayonnement émis à partir du collimateur et de réaliser le traitement informatique des clichés.

Le statif 31 est mobile en rotation à 360° autour d'un axe horizontal, ledit axe horizontal passant sensiblement par le milieu de la partie dans le plan vertical du statif 31, l'angle de rotation nul du statif 31 correspondant à la position verticale du statif 31.

Le collimateur comporte à l'intérieur de celui-ci deux bancs de lames (non représentés sur la Figure 4) permettant de décrire une fente glissante (« picket fence ») grâce à une translation transversale simultanée de ceux-ci, la translation transversale étant coordonnée avec la rotation du statif 31. Ainsi, le collimateur peut n'émettre qu'une partie du rayonnement de la tête d'irradiation 33 vers l'imageur de sortie 34 par l'intermédiaire des deux bancs de lames.

Lorsque le statif 31 est en rotation, l'imageur de sortie 34 a également la possibilité d'être déplacé dans les trois dimensions de l'espace grâce à des moteurs, des engrenages et des vis sans fin (non représentés sur la Figure 4).

L'imageur de sortie 34 est principalement utilisé pour réaliser des images planaires d'un patient en positionnement sur le support patient 32. Ces images permettent, avant de traiter le patient, de contrôler sa position par rapport au faisceau de traitement. Dans ce cas, l'imageur de sortie 34 reçoit les rayons provenant du collimateur et qui ont traversé le patient.

L'imageur de sortie 34 peut également être utilisé en tant qu'instrument de mesure du rayonnement émis par l'appareil de traitement par radiothérapie externe 30 afin de contrôler les performances de cet appareil (contrôles de qualité). Dans ce cas l'image est formée soit avec faisceau nu, soit avec un faisceau ayant traversé un objet-test.

Dans les deux cas, toutes les analyses réalisées par l'imageur de sortie 34 sur les images reposent en premier lieu sur des mesures de distance dans les images.

L'appareil de traitement par radiothérapie externe 30 comporte en outre une paire de glissières 35 disposées au niveau de la sortie du collimateur de la tête d'irradiation 33, un objet-test 1, 10, 20 pouvant être inséré dans ladite paire de glissières 35 de telle sorte que l'objet-test 1, 10, 20 soit disposé au plus près de la tête d'irradiation 33 de l'appareil de traitement par radiothérapie externe 30, l'objet-test 1, 10, 20 étant fermement fixé une fois inséré dans la paire de glissières 35 par l'intermédiaire de vis de telle sorte que l'objet-test 1, 10, 20 ne bouge pas par rapport à la source de rayonnement lorsque le statif 31 est en rotation.

L'imageur de sortie 34 peut être utilisé selon trois modes de fonctionnement parmi un mode statique dans lequel l'image est acquise par l'imageur de sortie 34 lorsque l'appareil de traitement par radiothérapie externe 30 est immobile, un mode cinéma dans lequel des images sont acquises par l'imageur de sortie 34 à intervalles réguliers pendant toute la rotation du statif 31 de l'appareil de traitement par radiothérapie externe 30, et un mode intégration dans lequel l'image acquise par l'imageur de sortie 34 correspond au cumul du rayonnement pendant toute la rotation du statif 31 de l'appareil de traitement par radiothérapie externe 30.

Dans les modes cinéma et intégration, les images acquises par l'imageur de sortie 34 présentent des artéfacts induits par les mouvements parasites de l'imageur de sortie 34 lorsque celui-ci est en mouvement avec la rotation du statif 31 de l'appareil de traitement par radiothérapie externe 30.

Afin de mesurer les mouvements parasites de l'imageur de sortie 34 par rapport à la source de rayonnement contenue dans la tête de l'appareil de traitement par radiothérapie externe afin de les compenser, un objet-test 1, 10, 20 est inséré dans la paire de glissières 35 disposées à la sortie du collimateur, l'objet-test 1, 10, 20 comprenant des éléments radio-opaques servant à mesurer les mouvements parasites verticaux, longitudinaux et transversaux de l'imageur de sortie 34 par rapport au collimateur lors de contrôles de performances du collimateur multi-lames utilisé en mode dynamique pour à la fois délimiter le faisceau d'irradiation et moduler son intensité durant le traitement.

Si l'on se réfère à la Figure 5, on peut voir qu'il y est représenté la superposition de la vue de dessus de l'objet-test 1 du premier mode de réalisation de la présente invention et de l'image associée capturée par l'imageur de sortie 34 de l'appareil de traitement par radiothérapie externe 30 à un angle de rotation de statif 31 donné.

L'objet-test 1 du premier (ou troisième) mode de réalisation de la présente invention peut être utilisé selon un procédé avec prétest de mesure et de correction des mesures réalisées dans les images correspondant aux tests de performance d'un appareil de traitement par radiothérapie externe 30 pour prendre en compte les mouvements parasites d'un imageur de sortie 34 équipant ledit appareil de traitement par radiothérapie externe 30 lorsque celui-ci est en mouvement, l'appareil de traitement par radiothérapie externe 30 comprenant en outre un statif 31 rotatif portant à une extrémité une tête d'irradiation 33 et à l'autre extrémité un imageur de sortie 34, comprenant les étapes consistant à :
- fixer un objet-test 1, 20 selon les premier ou troisième modes de réalisation sur la tête d'irradiation 33 de l'appareil de traitement par radiothérapie externe 30, l'objet-test 1, 20 étant fixé très fermement à une certaine distance de la source de rayonnement de la tête d'irradiation 33 et au plus près de celle-ci ;
- réaliser un prétest consistant à :
   - irradier l'objet-test 1, 20 avec un faisceau de rayonnement émis à partir de la tête d'irradiation 33 de l'appareil de traitement par radiothérapie externe 30 pendant une séquence d'angles de rotation du statif 31 de l'appareil de traitement par radiothérapie externe 30 ;
   - acquérir une succession d'images de l'objet-test 1, 20 sur l'imageur de sortie 34 en fonction de la séquence d'angles de rotation du statif 31 de l'appareil de traitement par radiothérapie externe 30 ;
   - mesurer la taille du motif géométrique formé par les billes 3a, 3b, 3c, 3d de l'objet-test 1, 20 et la position du centre dudit motif dans toutes les images acquises, à l'aide des marques créées par les billes 3a, 3b, 3c, 3d sur les images après irradiation de l'objet-test 1, 20 ;
   - comparer la taille réelle du motif géométrique formé par les billes 3a, 3b, 3c, 3d de l'objet-test 1, 20 à la taille mesurée du motif géométrique formé par les billes 3a, 3b, 3c, 3d de l'objet-test 1, 20 sur chaque image afin d'obtenir la distance source de rayonnement-imageur de sortie DSI et donc la mesure des mouvements verticaux parasites de l'imageur de sortie 34 en fonction de l'angle de rotation du statif 31 ; et
   - comparer la position du centre de l'imageur de sortie 34 à la position mesurée du centre du motif géométrique formé par les billes 3a, 3b, 3c, 3d de l'objet-test 1, 20 sur chaque image afin d'obtenir la mesure des mouvements longitudinaux et transversaux parasites de l'imageur de sortie 34 en fonction de l'angle de rotation du statif 31, la mesure étant corrigée des variations de DSI calculées précédemment ;
- retirer l'objet-test 1, 20 de la tête d'irradiation 33 ;
- acquérir les images correspondant au test de performance de l'appareil de traitement par radiothérapie externe ; et
- corriger les mesures réalisées dans ces images et caractérisant les performances de l'appareil de traitement par radiothérapie externe, de tous les mouvements parasites de l'imageur de sortie 34 à l'aide des mesures réalisées lors du prétest.

Il est à noter que ce procédé avec prétest n'est valable que dans le cas où les mouvements parasites de l'imageur de sortie 34 sont reproductibles entre le prétest et le test.

La Figure 5 représente la superposition de la vue de dessus de l'objet-test 1 du premier mode de réalisation de la présente invention et de l'image associée capturée par l'imageur de sortie 34 de l'appareil de traitement par radiothérapie externe 30 à un angle de rotation de statif 31 donné lors du procédé avec prétest.

Tel que mentionné précédemment, les éléments radio-opaques 3a, 3b, 3c, 3d de l'objet-test 1 forment un carré 4, le centre du carré étant représenté de manière illustrative dans la Figure 5 par une croix 40. Après irradiation de l'objet-test 1 par la tête d'irradiation 33, l'imageur de sortie 34 acquière des images 41a, 41b, 41c, 41d des éléments radio-opaques 3a, 3b, 3c, 3d, les images 41a, 41b, 41c, 41d des éléments radio-opaques 3a, 3b, 3c, 3d formant un carré 42 dont la taille est différente de celle du carré 4 (dans la Figure 5, les images 41a, 41b, 41c, 41d des éléments radio-opaques 3a, 3b, 3c, 3d et le carré 42 étant déplacés afin d'être centrés sur le centre de la croix 40 pour faciliter la compréhension), la différence de taille entre le carré 4 et le carré 42 permettant de mesurer la distance source de rayonnement-imageur de sortie (appelée DSI) et donc la mesure des mouvements verticaux parasites d_{V} de l'imageur de sortie 34 en fonction de l'angle de rotation du statif 31.

Ensuite, le centre du carré formé par les images 41a, 41b, 41c, 41d des éléments radio-opaques 3a, 3b, 3c, 3d est repéré sur la Figure 5 par une croix 43, la différence de position entre le centre de l'imageur de sortie 34 et le centre de la croix 43 après correction de la variation de DSI permettant de mesurer les mouvements longitudinaux parasites d_{L} et les mouvements transversaux parasites d_{T} de l'imageur de sortie 34 en fonction de l'angle de rotation du statif 31.

Si l'on se réfère à la Figure 6, on peut voir qu'il y est représenté la superposition de la vue de dessus de l'objet-test 10 du second mode de réalisation de la présente invention et de l'image associée capturée par l'imageur de sortie 34 de l'appareil de traitement par radiothérapie externe 30 à un angle de rotation de statif 31 donné.

L'objet-test 10 du second (ou troisième) mode de réalisation de la présente invention peut être utilisé selon un procédé sans prétest de mesure et de correction des mesures réalisées dans les images correspondant aux tests de performance d'un appareil de traitement par radiothérapie externe 30 pour prendre en compte les mouvements parasites d'un imageur de sortie 34 équipant ledit appareil de traitement par radiothérapie externe 30 lorsque celui-ci est en mouvement, l'appareil de traitement par radiothérapie externe 30 comprenant en outre un statif 31 rotatif portant à une extrémité une tête d'irradiation 33 qui se termine par un collimateur permettant de délimiter un faisceau de rayonnement et à l'autre extrémité l'imageur de sortie 34, caractérisé par le fait qu'il comprend les étapes consistant à :
- fixer un objet-test 10, 20 sur la tête d'irradiation 33 de l'appareil de traitement par radiothérapie externe 30, l'objet-test 10, 20 étant fixé très fermement à une certaine distance de la source de rayonnement de la tête d'irradiation 33 et au plus près de celle-ci ;
- irradier l'objet-test 10, 20 lors du test de performance de l'appareil traitement par radiothérapie externe avec un faisceau de rayonnement émis à partir de la tête d'irradiation 33 de l'appareil de traitement par radiothérapie externe 30 pendant une séquence d'angles de rotation du statif 31 de l'appareil de traitement par radiothérapie externe 30, le collimateur comportant une fente glissante glissant de manière transversale réalisée par deux bancs de lames afin de n'irradier qu'une seule colonne de billes des deux rangées de billes parallèles 11a, 11b pour un angle de rotation donné du statif 31 ;
- acquérir l'image de l'objet-test 10,20 sur l'imageur de sortie 34 utilisé en mode intégration pendant toute la séquence d'angles de rotation du statif 31, chaque colonne de l'image ayant été acquise pour un angle de rotation du statif 31 donné ;
- mesurer dans l'image acquise la distance longitudinale séparant dans chaque colonne de l'image la paire de billes 51a, 51b correspondant à l'angle de rotation de statif 31 associé et la position du centre de la paire de billes 51a, 51b correspondant à l'angle de rotation de statif 31 associé, à l'aide des marques créées par les billes des deux rangées de billes parallèles 11a, 11b sur l'image après irradiation de l'objet-test 10, 20 ;
- dans l'image acquise, comparer la distance réelle séparant les deux rangées de billes 11a, 11b à la distance mesurée séparant la paire de billes 51a, 51b correspondant à l'angle de rotation de statif 31 associé, afin d'obtenir la distance source de rayonnement-imageur de sortie (DSI) et donc la mesure des mouvements verticaux parasites de l'imageur de sortie 34 en fonction de l'angle de rotation du statif 31 ;
- dans l'image acquise, comparer la position réelle du centre de la paire de billes 51a, 51b correspondant à l'angle de rotation de statif 31 associé à la position mesurée du centre de la paire de billes 51a, 51b correspondant à l'angle de rotation de statif 31 associé, afin d'obtenir la mesure des mouvements longitudinaux et transversaux parasites de l'imageur de sortie 34 en fonction de l'angle de rotation du statif 31, la mesure étant corrigée des variations de DSI calculées précédemment ; et
- dans l'image acquise, correspondant également au test de performance de l'appareil de traitement par radiothérapie externe, corriger les mesures réalisées dans l'image et caractérisant les performances de l'appareil de traitement par radiothérapie externe, de tous les mouvements parasites de l'imageur de sortie 34 à l'aide des mesures réalisées précédemment.

Il est à noter que ce procédé sans prétest est valable même dans le cas où les mouvements parasites de l'imageur de sortie 34 ne sont pas reproductibles.

La Figure 6 représente la superposition de la vue de dessus de l'objet-test 10 du second mode de réalisation de la présente invention et de l'image associée capturée par l'imageur de sortie 34 de l'appareil de traitement par radiothérapie externe 30 à un angle de rotation de statif 31 donné, lors du procédé sans prétest.

Tel que mentionné précédemment, les éléments radio-opaques de l'objet-test 10 sont deux rangées parallèles de billes 11a, 11b. Pour chaque angle de statif 31, les deux bancs de lames 50 du collimateur sont positionnés afin de n'irradier qu'une seule paire de billes 51a, 51b des deux rangées de billes 11a, 11b. Après irradiation de l'objet-test 10 par la tête d'irradiation 33, l'imageur de sortie 34 acquière des images 52a, 52b de la paire de billes 51a, 52b, la distance longitudinale entre les images 52a, 52b de la paire de billes 51a, 51b étant différente de celle entre les billes de la paire de billes 51a, 51b (dans la Figure 6, les images 52a, 52b de la paire de billes 51a, 51b étant déplacées entre la paire de billes 51a, 51b afin de faciliter la compréhension), la différence de distance longitudinale entre les images 52a, 52b de la paire de billes 51a, 51b et les billes de la paire de billes 51a, 51b permettant de mesurer la distance source de rayonnement-imageur de sortie (DSI) et donc la mesure des mouvements verticaux parasites d_{V} de l'imageur de sortie 34 en fonction de l'angle de rotation du statif 31.

Ensuite, les centres de la paire de billes 51a, 51b et des images 52a, 52b de la paire de billes 51a, 51b sont repérés sur la Figure 6 respectivement par une croix 53 et une croix 54, la différence de position entre le centre de la croix 53 et le centre de la croix 54 après correction de la variation de DSI permettant de mesurer les mouvements longitudinaux parasites d_{L} et les mouvements transversaux parasites d_{T} de l'imageur de sortie 34 en fonction de l'angle de rotation du statif 31.

Il est à noter que l'objet-test 20 du troisième mode de réalisation de la présente invention peut soit être utilisé selon le procédé avec prétest en utilisant les quatre éléments radio-opaques 3a, 3b, 3c, 3d, soit être utilisé selon le procédé sans prétest en utilisant les deux rangées de billes 11a, 11b.

La correction des mesures réalisées dans les images correspondant aux tests de performance pour prendre en compte les mouvements parasites de l'imageur de sortie 34 pendant l'acquisition de l'image peut être réalisée selon deux méthodes qui sont une méthode de correction intégrale de l'image des trois types de mouvements parasites (transversal, longitudinal et vertical) de l'imageur de sortie 34 et une méthode de correction uniquement des variations parasites de DSI (mouvement parasite vertical) des mesures de distance ou d'intensité réalisées dans les images non corrigées.

La méthode de correction intégrale n'est possible que sur des images qui se forment transversalement au fur et à mesure de la rotation du statif 31 grâce à la progression transversale d'une fente glissante délimitée par les deux bancs de lames 50 du collimateur. Dans cette méthode, tous les pixels d'une colonne de ce type d'image étant acquis pour un même angle de rotation de statif 31, la correction de l'image consiste à déplacer transversalement et longitudinalement la colonne de pixels en fonction de l'angle du statif 31, les déplacements mesurés devant être préalablement remis à l'échelle en appliquant le facteur DSI nominale / DSI. Par ailleurs, l'intensité de chaque pixel de la colonne de pixels est pour sa part corrigée de la loi de l'inverse du carré de la distance à la source de rayonnement, c'est-à-dire l'inverse du carré de la DSI correspondant à l'angle de rotation de statif 31 associé à la colonne de l'image à laquelle appartient le pixel, cette correction d'intensité étant nécessaire pour tenir compte du fait que si l'imageur de sortie 34 s'éloigne la quantité de rayons X parvenant à l'imageur de sortie 34 diminue selon l'inverse du carré de la distance par rapport à la source de rayonnement. Enfin, l'image résultante correspond à une resegmentation sur la trame de pixels initiale des colonnes de pixels déplacées.

La méthode de correction uniquement des variations parasites de DSI n'est pas une correction d'images mais une correction des mesures de distance ou d'intensité de signal réalisées dans les images non corrigées. Cette méthode de correction partielle est applicable et nécessaire : i) lorsque l'analyse d'une image acquise lors de la rotation du statif 31 ne repose que sur des comparaisons d'intensité de signal entre différentes régions de l'image acquises à différents angles de rotation de statif 31, ii) lorsque l'analyse consiste à comparer des niveaux de signaux entre différentes images acquises à différents angles fixes de rotation de statif 31, et iii) lorsque l'analyse consiste à comparer des distances par rapport au centre de faisceau mesurées sur différentes images acquises lorsque le statif 31 est fixe mais pour des angles de rotation du statif 31 différents. Dans les cas i) et ii), on corrige l'intensité des signaux mesurés en les ramenant tous à la DSI nominale à l'aide de la loi de l'inverse du carré de la distance à la source de rayonnement, et dans le cas iii), on ramène toutes les distances à la DSI nominale en appliquant la correction suivante sur les distances mesurées : (distance corrigée à la DSI nominale) = (distance mesurée à la DSI) * (DSI nominale / DSI).

## Revendications

1. Objet-test (1; 10; 20) pour un appareil de traitement par radiothérapie externe (30), ledit appareil de traitement par radiothérapie externe (30) comprenant un statif (31) rotatif portant à une extrémité une tête d'irradiation (33) contenant une source de rayonnement et à l'autre extrémité un imageur de sortie (34) de type imageur portal, ledit objet-test (1; 10; 20) étant destiné à corriger les mouvements parasites de l'imageur de sortie (34), vis-à-vis de la source de rayonnement, lors d'un mouvement de l'appareil de traitement par radiothérapie externe (30), **caractérisé par le fait que** ledit objet-test (1; 10; 20) comprend une plaque radiotransparente (2) dans l'épaisseur de laquelle est noyé au moins un élément radio-opaque (3a, 3b, 3c, 3d; 11a, 11b), ledit ou lesdits éléments radio-opaques formant un motif géométrique ayant au moins deux points et le centre dudit motif géométrique correspondant au centre de ladite plaque radiotransparente (2), des moyens de fixation permettant la fixation de l'objet-test (1; 10; 20) sur la tête d'irradiation (33), et **par le fait que** la plaque radiotransparente (2) porte deux rangées parallèles de billes 11a, 11b), chaque rangée de billes 11a, 11b) comprenant au moins deux billes, les deux rangées de billes 11a, 11b) comprenant le même nombre de billes espacées du même pas, de telle sorte que la ligne joignant les billes en regard des deux rangées est perpendiculaire à la direction des rangées de billes (11a, 11b).

2. Objet-test (20) selon la revendication 1, **caractérisé par le fait qu'**il comprend en outre au moins quatre billes radio-opaques (3a, 3b, 3c, 3d) formant un carré ou un rectangle dont le contour entoure les deux rangées de billes 11a, 11b), le centre du carré ou rectangle formé par les quatre billes radio-opaques (3a, 3b, 3c, 3d) correspondant au barycentre des deux rangées de billes 11a, 11b).

3. Objet-test (1, 10, 20) selon l'une quelconque des revendications 1 ou 2, **caractérisé par le fait que** la plaque radiotransparente (2) est en matière plastique radio-transparente, de préférence du polycarbonate.

4. Objet-test (1, 10, 20) selon l'une quelconque des revendications 1 à 3, **caractérisé par le fait que** l'épaisseur de la plaque radiotransparente (2) est constante et comprise entre 3 et 10 mm, de préférence 6 mm.

5. Objet-test (1, 10, 20) selon l'une quelconque des revendications 1 à 4, **caractérisé par le fait que** les éléments radio-opaques (3a, 3b, 3c, 3d, 11a, 11b) sont en carbure de tungstène.

6. Objet-test (1, 20) selon l'une des revendications 1 à 5, **caractérisé par le fait que** chaque bille radio-opaque est noyée dans la plaque radiotransparente (2) de telle sorte que son centre soit à une distance de la face de la plaque radiotransparente (2) sensiblement égale à son rayon.

7. Objet-test (1, 10, 20) selon l'une quelconque des revendications 1 à 6, **caractérisé par le fait que** la plaque radiotransparente (2) comporte un réticule (9) gravé au niveau de la face de celle-ci destinée à être en regard de la tête d'irradiation (33), ledit réticule (9) représentant le centre de la plaque radiotransparente (2).

8. Procédé avec prétest de mesure et de correction des mesures réalisées dans les images correspondant aux tests de performance d'un appareil de traitement par radiothérapie externe (30) pour prendre en compte les mouvements parasites d'un imageur de sortie (34) équipant ledit appareil de traitement par radiothérapie externe (30), vis-à-vis de la source de rayonnement de l'appareil de traitement par radiothérapie externe (30) lorsque celui-ci est en mouvement, l'appareil de traitement par radiothérapie externe (30) comprenant en outre un statif (31) rotatif portant à une extrémité une tête d'irradiation (33) contenant la source de rayonnement et à l'autre extrémité un imageur de sortie (34) de type imageur portal, **caractérisé par le fait qu'**il comprend les étapes consistant à :
- fixer un objet-test (1, 20) tel que défini à l'une quelconque des revendications 1 à 7 sur la tête d'irradiation (33) de l'appareil de traitement par radiothérapie externe (30), l'objet-test (1, 20) étant fixé très fermement à une certaine distance de la source de rayonnement de la tête d'irradiation (33) et au plus près de celle-ci ;
- réaliser un prétest consistant à :
- irradier l'objet-test (1, 20) avec un faisceau de rayonnement émis à partir de la tête d'irradiation (33) de l'appareil de traitement par radiothérapie externe (30) pendant une séquence d'angles de rotation du statif (31) de l'appareil de traitement par radiothérapie externe (30) ;
- acquérir une succession d'images de l'objet-test (1, 20) sur l'imageur de sortie (34) en fonction de la séquence d'angles de rotation du statif (31) de l'appareil de traitement par radiothérapie externe (30) ;
- mesurer la taille du motif géométrique formé par les billes (3a, 3b, 3c, 3d) de l'objet-test (1, 20) et la position du centre dudit motif dans toutes les images acquises, à l'aide des marques créées par les billes (3a, 3b, 3c, 3d) sur les images après irradiation de l'objet-test (1, 20) ;
- comparer la taille réelle du motif géométrique formé par les billes (3a, 3b, 3c, 3d) de l'objet-test (1, 20) à la taille mesurée du motif géométrique formé par les billes (3a, 3b, 3c, 3d) de l'objet-test (1, 20) sur chaque image afin d'obtenir la distance source de rayonnement-imageur de sortie (DSI) et donc la mesure des mouvements verticaux parasites de l'imageur de sortie (34) en fonction de l'angle de rotation du statif (31) ; et
- comparer la position du centre de l'imageur de sortie (34) à la position mesurée du centre du motif géométrique formé par les billes (3a, 3b, 3c, 3d) de l'objet-test (1, 20) sur chaque image afin d'obtenir la mesure des mouvements longitudinaux et transversaux parasites de l'imageur de sortie (34) en fonction de l'angle de rotation du statif (31), la mesure étant corrigée des variations de DSI calculées précédemment ;
- retirer l'objet-test (1, 20) de la tête d'irradiation (33) ;
- acquérir les images correspondant au test de performance de l'appareil de traitement par radiothérapie externe ; et
- corriger les mesures réalisées dans ces images et caractérisant les performances de l'appareil de traitement de radiothérapie externe de tous les mouvements parasites de l'imageur de sortie (34) à l'aide des mesures réalisées lors du prétest.

9. Procédé sans prétest de mesure et de correction des mesures réalisées dans les images correspondant aux tests de performance d'un appareil de traitement par radiothérapie externe (30) pour prendre en compte les mouvements parasites d'un imageur de sortie (34) équipant ledit appareil de traitement par radiothérapie externe (30) vis-à-vis de la source de rayonnement de l'appareil de traitement par radiothérapie externe (30) lorsque celui-ci est en mouvement, l'appareil de traitement par radiothérapie externe (30) comprenant en outre un statif (31) rotatif portant à une extrémité une tête d'irradiation (33) contenant la source de rayonnement et qui se termine par un collimateur comprenant deux bancs transversalement opposés portant chacun de multiples lames permettant de délimiter un faisceau de rayonnement de forme complexe et à l'autre extrémité un imageur de sortie (34) de type imageur portal, **caractérisé par le fait qu'**il comprend les étapes consistant à :
- fixer un objet-test (10, 20) tel que défini à l'une quelconque des revendications 1 à 7 sur la tête d'irradiation (33) de l'appareil de traitement par radiothérapie externe (30), l'objet-test (10, 20) étant fixé très fermement à une certaine distance de la source de rayonnement de la tête d'irradiation (33) et au plus près de celle-ci ;
- irradier l'objet-test (10, 20) lors du test de performance de l'appareil de traitement par radiothérapie externe avec un faisceau de rayonnement émis à partir de la tête d'irradiation (33) de l'appareil de traitement par radiothérapie externe (30) pendant une séquence d'angles de rotation du statif (31) de l'appareil de traitement par radiothérapie externe (30), le collimateur délimitant une fente glissante glissant de manière transversale réalisée par ses deux bancs de lames transversalement opposés afin de n'irradier qu'une seule colonne de billes des deux rangées de billes parallèles 11a, 11b) pour un angle de rotation donné du statif (31) ;
- acquérir l'image de l'objet-test (10, 20) sur l'imageur de sortie (34) pendant toute la séquence d'angles de rotation du statif (31), chaque colonne de l'image ayant été acquise pour un angle de rotation du statif (31) donné ;
- mesurer dans l'image acquise la distance longitudinale séparant dans chaque colonne de l'image la paire de billes (51a, 51b) correspondant à l'angle de rotation de statif (31) associé et la position du centre de la paire de billes (51a, 51b) correspondant à l'angle de rotation de statif (31) associé, à l'aide des marques créées par les billes des deux rangées de billes parallèles 11a, 11b) sur l'image après irradiation de l'objet-test (10, 20) ;
- dans l'image acquise, comparer la distance réelle séparant les deux rangées de billes 11a, 11b) à la distance mesurée séparant la paire de billes (51a, 51b) correspondant à l'angle de rotation de statif (31) associé, afin d'obtenir la distance source de rayonnement-imageur de sortie (DSI) et donc la mesure des mouvements verticaux parasites de l'imageur de sortie (34) en fonction de l'angle de rotation du statif (31) ;
- dans l'image acquise, comparer la position réelle du centre de la paire de billes (51a, 51b) correspondant à l'angle de rotation de statif (31) associé à la position mesurée du centre de la paire de billes (51a, 51b) correspondant à l'angle de rotation de statif (31) associé, afin d'obtenir la mesure des mouvements longitudinaux et transversaux parasites de l'imageur de sortie (34) en fonction de l'angle de rotation du statif (31), la mesure étant corrigée des variations de DSI calculées précédemment ; et
- dans l'image acquise, correspondant également au test de performance de l'appareil de traitement par radiothérapie externe, corriger les mesures réalisées dans l'image et caractérisant les performances de l'appareil de traitement par radiothérapie externe, de tous les mouvements parasites de l'imageur de sortie (34) à l'aide des mesures réalisées précédemment.
